# EUROPEAN PATENT APPLICATION

(11) **EP 4 272 960 A1**
(43) Date of publication of application: **08.11.2023**
(21) Application number: 22020208.9
(22) Date of filing: 06.05.2022
(51) Int. Cl.: B33Y 10/00, B33Y 70/00, B33Y 80/00, C08L 83/04, C08L 83/08

(54) **METHOD FOR ADDITIVE MANUFACTURING OF A 3D ARTICLE**

(71) Applicant: ELKEM SILICONES France SAS, 69190 SAINT-FONS (FR)
(72) Inventor: FRANCES, Jean-Marc, 69330 MEYZIEU (FR); PERRINET, Clément, 69100 VILLEURBANNE (FR)
(74) Representative: Menville, Laure

(57) **Abstract**

The invention relates to a method for additive manufacturing of a 3D article with a material jetting 3D printer, using a specific silicone composition, curable by thiol-ene reaction, and having a viscosity at 70°C of less than 100 mPa.s. Said curable silicone composition comprises at least one cyclic organopolysiloxane compound A having between 3 and 8 silicon atoms and at least two C2-C8 alkenyl radicals bonded to silicon atoms per molecule, or at least one cyclic organopolysiloxane compound A' having between 3 and 8 silicon atoms and at least two (meth)acrylate groups per molecule, at least one mercapto-containing organopolysiloxane compound B comprising, per molecule, at least two mercapto group, at least one photoinitiator C, and optionally at least one filler D. The invention further relates to use of said curable silicone composition as an ink for a method for additive manufacturing of a 3D article with a material jetting 3D printer.

## Description

### Technical field

The present disclosure relates to a method of additive manufacture of a three-dimensional (3D) article made at least partially of a cured silicone material. More specifically, it relates to a method for additive manufacturing of a 3D article comprising a cured silicone material with a material jetting 3D printer.

### Background of the invention

Three-dimensional (3D) printing is a process in which material can be deposited, joined or solidified under computer control to create a three-dimensional object with material being added together layer by layer. Additive manufacturing techniques covers different technologies in which materials are accumulated layer by layer via specific techniques such as extrusion, sintering, melting, photopolymerization, jetting, lamination and deposition.

Silicone has been used for a variety of medical applications mainly owing to its biocompatibility and thermal stability. Medical-grade silicones are used in anatomic models, mannequins, prosthetics, pharmaceuticals, lubricating injection devices etc. Producing silicone structures by 3D printing is a promising method with high design flexibility.

However, the industry is still struggling with the problem of the printability of the silicone compositions because silicone compositions are particularly viscous. Silicone compositions are most commonly printed according to the material extrusion technology: it is defined as an additive manufacturing process in which material, here a silicone composition, is selectively dispensed through a nozzle or orifice. Utilizing the shear thinning behavior of some high viscosity compositions during the printing process has been proposed for example in the patent application WO 2015/069454. It discloses a shear-thinning UV-curable silicone composition, which viscosity decreases as a shear applied to the composition increases. It is suggested that the shear-thinning property of the composition can make the composition suitable for use with a variety of methods, such as printing methods. However, shear thinning behavior does not eliminate the rheology constraint for a successful printing.

Inkjet printing technology has seen enormous development as a precision microdispensing tool. Traditionally inkjet printing has been mostly applied in the fabrication of 2D patterns or structures due to the requirement of low viscosity of inks. Viscosities of inks for traditional inkjet system is normally in the range of about 1-40 mPa.s. Some academic teams and companies developed droplet deposition systems (typically with piezoelectric microdispenser or dual piezoelectric/pneumatic mechanism) which allow the 3D printing of silicone formulations having viscosities as high as 100,000 mPa.s (for instance Yang, H., et al. "High viscosity jetting system for 3D reactive inkjet printing." 2013 International Solid Freeform Fabrication Symposium. University of Texas at Austin, 2013). However, even though these technologies may be referenced as material jetting or inkjet, they request specific printheads and do not lead to the same resolution of the printing as standard 2D inkjet printing devices. Traditional inkjet printing is well known for its high resolution (around ten picolitres) and its velocity compared to other printing processes. It would be advantageous to develop a silicone inkjet 3D printing process which uses the same technology as the conventional 2D inkjet printing, and preferably the same printheads.

The patent application WO 2020/051039 discloses a composition for forming silicone elastomer especially suited for inkjet printing based on its low viscosity relative to conventional compositions that may be of higher viscosity and thus are too thick to be printed using conventional inkjet printers and methodologies. Said composition is a hydrosilylation-curable silicone composition. This type of chemistry requires too long and impractical crosslinking times and/or require high temperatures, thus extra heating devices and costs.

In view of the current situation, it would be advantageous to provide an improved formulation of a curable silicone composition, which would be suitable as an ink to be used in an inkjet 3D printing process. 3D printed articles obtained by such inkjet 3D printing process could advantageously be used for medical applications, like medical materials and/or devices.

### Summary of the invention

As a result of diligent research, the inventors of the present invention found that it was possible to solve the above-mentioned problems by using a specific silicone composition, curable by thiol-ene reaction, and having some specific viscosity requirements:
A method for additive manufacturing of a 3D article is provided, said method comprising the steps of:
1) printing a first curable silicone composition on a substrate with a material jetting 3D printer and exposing to radiation to form a first layer,
2) printing a second curable silicone composition on the first or previous layer with said material jetting 3D printer and exposing to radiation to form a subsequent layer; and
3) optionally repeating step 2) with independently selected curable silicone composition for any additional layer needed;
   wherein at least one of said curable silicone compositions is a curable silicone composition X, wherein said curable silicone composition **X** has a viscosity at 70°C of less than 100 mPa.s, and said curable silicone composition **X** comprises:
   (A) at least one cyclic organopolysiloxane compound **A** having between 3 and 8 silicon atoms and at least two C₂-C₈ alkenyl radicals bonded to silicon atoms per molecule, or at least one cyclic organopolysiloxane compound **A'** having between 3 and 8 silicon atoms and at least two (meth)acrylate groups per molecule
   (B) at least one mercapto-containing organopolysiloxane compound **B** comprising, per molecule, at least two mercapto group,
   (C) at least one photoinitiator **C**, and
   (D) optionally at least one filler **D**.

The present invention further relates to use of a curable silicone composition **X**, which has a viscosity at 70°C of less than 100 mPa.s, and which comprises:
(A) at least one cyclic organopolysiloxane compound **A** having between 3 and 8 silicon atoms and at least two C₂-C₈ alkenyl radicals bonded to silicon atoms per molecule, or at least one cyclic organopolysiloxane compound **A'** having between 3 and 8 silicon atoms and at least two (meth)acrylate groups per molecule,
(B) at least one mercapto-containing organopolysiloxane compound **B** comprising, per molecule, at least two mercapto group,
(C) at least one photoinitiator **C**, and
(D) optionally at least one filler **D**,
   as an ink for a method for additive manufacturing of a 3D article with a material jetting 3D printer.

### Detailed description of the invention

The present invention relates to a method for additive manufacturing of a 3D article. Said method according to the present invention comprises at least the following steps:
1) printing a first curable silicone composition on a substrate with a material jetting 3D printer and exposing to radiation to form a first layer,
2) printing a second curable silicone composition on the first or previous layer with said material jetting 3D printer and exposing to radiation to form a subsequent layer; and
3) optionally repeating step 2) with independently selected curable silicone composition for any additional layer needed

According to ISO/ASTM Standard 52900 ("Additive manufacturing - General principles - Terminology", 2017), "additive manufacturing (AM)" is defined as a process of joining materials to make parts from 3D model data, usually layer upon layer, as opposed to subtractive manufacturing and formative manufacturing methodologies. Synonyms associated with AM include additive fabrication, additive processes, additive techniques, additive layer manufacturing, layer manufacturing, solid freeform fabrication, and freeform fabrication. Besides, "3D printing" is defined as the fabrication of objects through the deposition of a material using a print head, nozzle, or another printer technology. It is a term often used in a non-technical context synonymously with additive manufacturing. As used herein, "3D printing" is generally interchangeable with "additive manufacturing" and vice versa. "3D printer" is defined as a machine used for 3D printing.

Still according to ISO/ASTM Standard 52900, "material jetting" refers to an additive manufacturing process in which droplets of build material are selectively deposited.

Material jetting 3D printers are similar to traditional paper printers, e.g. inkjet printers. In material jetting, a print head moves around a print area jetting the particular composition. Repeating this process builds up the object one layer at a time. The material jetting 3D printer according to the invention typically comprises at least one printing head having a plurality of inkjet type nozzles. The curable silicone composition according to the invention is jetted through said inkjet type nozzles in the form of individual droplets, discontinuously, at the desired location of the work plane. Material jetting 3D printer may comprise preferably 2 to 200 inkjet type nozzles, allowing the site-selective application where appropriate of a curable silicone composition. The nozzles can be positioned accurately in x-, y- and z-directions to permit precisely targeted deposition of drops of the curable silicone composition. Commercial examples of printheads are those provided by the company Xaar, for example Xaar 1002 printhead range, or those provided by the company RICOH, for example RICOH MH5420/5440 printhead.

Each nozzle is connected to one or a plurality of reservoirs that are subjected to pressure and connected via a metering line. The curable silicone composition according to the invention may be supplied in one container or cartridge or in two or more containers or cartridges. If the curable silicone composition is a mono-component composition, then it may contain advantageously sufficient amount of suitable curing inhibitor to prevent any unwanted curing within the container or cartridge. If the curable silicone composition is a multi-part curable composition, then the multiple composition parts provided in two or more containers or cartridges may be homogeneously mixed upstream or downstream of the reservoir(s). Alternatively, each part of the multi-part composition may be jetted separately by dedicated nozzle(s), and the mixing of the multiple parts may occur outside of the nozzle(s), directly on the support.

The support according to the present invention refers to the material located in the bottom of the 3D printer and which is provided for carrying the 3D article obtained by additive manufacturing. One function of the support is helping in preventing collapse during the printing process. Supports can help to prevent part deformation, secure a part to the printing bed and ensure that parts are attached to the main body of the printed part. A suitable support may be selected by the person skilled in the art according to the common knowledge of the technical field. Some examples could be found in the patent applications US 2015/0028523, US 2018/0036953 and WO 2020/127882.

At the time of being jetted by the material jetting 3D printer according to the invention, the curable silicone composition can be at a temperature comprised between 20°C and 80°C. According to one embodiment, the temperature is room temperature, typically between 15°C and 30°C, preferably around 25°C. According to another embodiment, the temperature in comprised between 50°C and 80°C. The temperature may be controlled by any means known by the person skilled in the art.

The curable silicone composition is exposed to radiation in order to cause the curing of the composition. Preferably, the radiation is a UV and/or a visible radiation. The term "UV" as used herein refers to ultraviolet light, which is electromagnetic radiation with a wavelength of about 100 nm to about 400 nm, thus below the visible light spectrum. In the context of UV, UV-A, UV-B and UV-C may be defined: UV-A has typically wavebands located between 315 nm and 400 nm, UV-B has typically wavebands located between 280 nm and 315 nm, and UV-C has typically wavebands located between 100 nm and 280 nm. Besides, a visible radiation has typically wavebands located between 400 nm and 800 nm.

In the method according to the present invention, the radiation has preferably a wavelength between 100 nm and 450 nm, more preferable between 200 nm and 420 nm, and even more preferably between 250 nm and 405 nm.

The radiation can be provided by a doped or non-doped mercury vapor lamp having a wavelength range from typically 100 nm to 450 nm. Alternatively, light sources providing UV or visible point light can be used, in particular LED. The term "LED" as used herein refers to Light-Emitting Diode. According to a preferred embodiment, the radiation is a UV-radiation provided by a UV-LED. Said UV-LED can emit a radiation with a wavelength of 365 nm, 385 nm, 395 nm or 405 nm.

Advantageously, the curable silicone composition according to the present invention can cure quickly and efficiently when exposed to suitable radiation. Therefore, the exposition to radiation can be very short, for example less than 10 second. Any suitable amount of radiation can be used to cure the composition, such as about 1 mJ/cm² to about 1 J/cm². The exposition to radiation during the printing of each layer may be either continuous or discontinuous. Jetting the curable silicone composition and exposing it to radiation can be done simultaneously or successively. Preferably, the exposition to radiation is provided by flash lightening with a frequency of preferably from 1000 Hz to 50000 Hz. The flashing frequency can be aligned with the speed of material jetting by the nozzles, so that material jetting and irradiating can be performed alternatively at very high speed.

In the method accord to the present invention, at least one layer is printed with a curable silicone composition **X** which comprises:
(A) at least one cyclic organopolysiloxane compound **A** having between 3 and 8 silicon atoms and at least two C₂-C₈ alkenyl radicals bonded to silicon atoms per molecule, or at least one cyclic organopolysiloxane compound **A'** having between 3 and 8 silicon atoms and at least two (meth)acrylate groups per molecule,
(B) at least one mercapto-containing organopolysiloxane compound **B** comprising at least two mercapto group per molecule,
(C) at least one photoinitiator **C**, and
(D) optionally at least one filler **D.**

Said curable silicone composition **X** can cure by thiol-ene reaction: a thiol group from organopolysiloxane compound **B** reacts either with an alkenyl radical from organopolysiloxane compound **A** or with an (meth)acrylate radical from organopolysiloxane compound **A'** based on thiol-ene reaction or on Michael Addition under appropriate reaction conditions to form a linkage -S-.

The organopolysiloxane compound **A** is a cyclic organopolysiloxane having between 3 and 8 silicon atoms and at least two C₂-C₈ alkenyl radicals bonded to silicon atoms per molecule. The organopolysiloxane **A** can be represented by the following formula:

[ WₐZ_{b}SiO_{2/2} ]ₘ [ Z₂SiO_{2/2} ]ₙ

wherein:
- the symbols W, which may be identical or different, represent a linear or branched C₂-C₈ alkenyl group;
- the symbols Z, which may be identical or different, represent a monovalent hydrocarbon-based group containing from 1 to 30 carbon atoms, optionally substituted by at least one halogen atom;
- a = 1 or 2, b = 0 or 1 and a+b = 2; and
- m ≥ 2, n ≥ 0, and 3 ≤ m+n ≤ 8.

Each Z, independently from each other, can preferably be selected from the group consisting of alkyl groups containing from 1 to 8 carbon atoms and aryl groups containing between 6 and 12 carbon atoms, optionally substituted by at least one halogen atom, and even more preferentially selected from the group consisting of methyl, ethyl, propyl, 3,3,3-trifluoropropyl, xylyl, tolyl and phenyl radicals. Advantageously, each Z, independently from each other, can be selected from the group consisting of methyl and phenyl radicals.

Each W, independently from each other, can preferably be selected from the group consisting of: vinyl, propenyl, 3-butenyl, 5-hexenyl, and 7-octenyl; and preferably, W is a vinyl.

In a preferred embodiment, a=1 and b=1.

According to a very preferred embodiment, the cyclic organopolysiloxane **A** is 1,3,5,7-tetramethyl-1,3,5,7-tetravinyl-cyclotetrasiloxane, which has the formula ((CH₃)(CH₂=CH)SiO_{2/2})₄. Said compound can also be named "D₄Vi" according to the common terminology of the technical field.

The content of alkenyl radical in the cyclic organopolysiloxane **A** according to the present invention is preferably between 4wt.% and 30wt.% (weight of the alkenyl radical group, of the total weight of the cyclic organopolysiloxane **A**).

The cyclic organopolysiloxane **A** according to the present invention shows advantageously a very low viscosity: the dynamic viscosity at 25°C is preferably comprised between 0.1 mPa.s and 500 mPa.s.

The content of the cyclic organopolysiloxane **A** can be from 5wt.% to 70wt.%, preferably from 10wt.% to 65wt.%, and even more preferably from 15 wt.% to 60wt.%, of the total weight of the curable silicone composition **X.**

The organopolysiloxane compound **A'** is a cyclic organopolysiloxane having between 3 and 8 silicon atoms and at least two (meth)acrylate groups per molecule. As used herein, "(meth)acrylate" group means acrylate group or methacrylate group. The organopolysiloxane **A'** can be represented by the following formula:

[ W'ₐZ_{b}SiO_{2/2} ]ₘ [ Z₂SiO_{2/2} ]ₙ

wherein:
- the symbols W', which may be identical or different, represent a (meth)acrylate group;
- the symbols Z, which may be identical or different, represent a monovalent hydrocarbon-based group containing from 1 to 30 carbon atoms, optionally substituted by at least one halogen atom;
- a = 1 or 2, b = 0 or 1 and a+b = 2; and
- m ≥ 2, n ≥ 0, and 3 ≤ m+n ≤ 8.

Each Z, independently from each other, can preferably be selected from the group consisting of alkyl groups containing from 1 to 8 carbon atoms and aryl groups containing between 6 and 12 carbon atoms, optionally substituted by at least one halogen atom, and even more preferentially selected from the group consisting of methyl, ethyl, propyl, 3,3,3-trifluoropropyl, xylyl, tolyl and phenyl radicals. Advantageously, each Z, independently from each other, can be selected from the group consisting of methyl and phenyl radicals.

Each W, independently from each other, can preferably be selected from the following general formulas:
-CₙH₂ₙO-CH₂CHR²(CH₂)ₘ-OCOCH=CHR³, wherein n is 3 or 4, m is 0 or 1, R² is H, OH or -C₂H_{2z}-CH₂OH, z is 0, 1, 2 or 3, and R³ is H or -CH₃, or
-CₙH₂ₙO-COCH=CHR³, wherein n is 3 or 4, and R³ is H or -CH₃.

In a preferred embodiment, a=1 and b=1.

According to a very preferred embodiment, the cyclic organopolysiloxane **A'** is 1,3,5,7-tetramethyl-1,3,5,7-tetra(acryloxypropyl)-cyclotetrasiloxane, which has the formula ((CH₃)(CH₂=CHC(O)-O-(CH₂)₃)SiO_{2/2})₄.

The content of (meth)acrylate radical in the cyclic organopolysiloxane **A'** according to the present invention is preferably between 4wt.% and 30wt.% (weight of the (meth)acrylate radical group, of the total weight of the cyclic organopolysiloxane **A'**).

The cyclic organopolysiloxane **A'** according to the present invention shows advantageously a very low viscosity: the dynamic viscosity at 25°C is preferably comprised between 10 mPa.s and 500 mPa.s.

The content of the cyclic organopolysiloxane **A** can be from 5wt.% to 70wt.%, preferably from 10wt.% to 65wt.%, and even more preferably from 15wt.% to 60wt.%, of the total weight of the curable silicone composition **X.**

The organopolysiloxane compound **B** according to the invention is a mercapto-functional polyorganosiloxane. Here, a "mercapto function", also known as a "thiol function", designates the chemical group -SH. A "mercapto functional" compound or "mercapto-functional" moiety refers respectively to any compound or moiety comprising a mercapto function.

The organopolysiloxane **B** can be a polyorganosiloxane compound comprising preferably:
at least two siloxy units of formula: R_{c}Z_{d}SiO(₄-_{c-d})_{/2}
wherein:
   - the symbols R^{(SH)}, which may be identical or different, represent a mercapto(C₁₋₃₀)hydrocarbyl group,
   - the symbols Z, which may be identical or different, represent a monovalent hydrocarbon-based group containing from 1 to 30 carbon atoms, optionally substituted by at least one halogen atom, and
   - c=1, 2 or 3, preferably c=1 or 2, more preferably c=1; d=0, 1 or 2; and c+d=1, 2 or 3; and optionally other siloxy units of formula: ZₑSiO_{(4-e)/2}
      wherein the symbols Z have the same definition as above, and e = 0, 1, 2, or 3.

Each Z, independently from each other, can preferably be selected from the group consisting of alkyl groups containing from 1 to 8 carbon atoms and aryl groups containing between 6 and 12 carbon atoms, optionally substituted by at least one halogen atom, and even more preferentially selected from the group consisting of methyl, ethyl, propyl, 3,3,3-trifluoropropyl, xylyl, tolyl and phenyl radicals. Advantageously, each Z, independently from each other, can be selected from the group consisting of methyl and phenyl radicals.

Preferably, the mercapto-hydrocarbyl group according to the invention is a hydrocarbyl group, preferably an alkyl group, that contains at least one mercapto function, preferably at the end of the alkyl chain opposite to where the alkyl group bonds to the silicon atom of the siloxane unit (i.e., a terminal thiol group). At each occurrence, the symbol R^{(SH)} can independently represent preferably a mercapto(C₁₋₃₀)alkyl group, and more preferably a mercapto(C₁₋₁₂) alkyl group. Even more preferably, R^{(SH)} can be selected from the group consisting of -CH₂SH, -CH₂CH₂SH, -CH₂CH₂CH₂SH, and-CH₂CH₂CH₂CH₂SH, and even more preferably R can represent the mercapto-propyl group (i.e.,-CH₂CH₂CH₂SH).

As used herein, the siloxy units "M", "D", "T", "Q", "D^{∗}", and "T^{∗}" are defined as follows:
- a siloxy unit M represents a siloxy unit of formula Z₃SiO_{1/2},
- a siloxy unit D represents a siloxy unit of formula Z₂SiO_{2/2},
- a siloxy unit T represents a siloxy unit of formula ZSiO_{3/2},
- a siloxy unit Q represents a siloxy unit of formula SiO_{4/2},
- a siloxy unit M^{∗} represents a siloxy unit of formula R^{(SH)}Z₂SiO_{1/2},
- a siloxy unit D^{∗} represents a siloxy unit of formula R^{(SH)}ZSiO_{2/2},
- a siloxy unit T^{∗} represents a siloxy unit of formula R^{(SH)}SiO_{3/2},
   wherein R^{(SH)} and Z have the same definitions as above.

The organopolysiloxane **B** can be linear, cyclic, or branched.

According to a first embodiment, the organopolysiloxane **B** can be linear. It can consist essentially of a combination of D and/or D^{∗} units, with terminal siloxy units M and/or M^{∗}. Examples of linear mercapto-functional organopolysiloxane **B** according to the present invention are:
- poly(dimethylsiloxane) with mercapto-functional silyl terminal groups;
- poly(dimethylsiloxane-co-methyl-mercapto-functional-siloxane) with trimethylsilyl terminal groups;
- poly(dimethylsiloxane-co- methyl-mercapto-functional-siloxane) with mercapto-functional silyl terminal group; and
- poly(methyl-mercapto-functional-siloxane) with trimethylsilyl terminal groups.

According to a second embodiment, the organopolysiloxane **B** can be cyclic. It can consist essentially of D^{∗} units with optionally D units. One examples of cyclic mercapto-functional organopolysiloxane **B** according to the present invention is a cyclic poly(methyl-mercapto-functional-siloxane).

According to a third embodiment, the organopolysiloxane **B** can be branched. The branched organopolysiloxane **B** comprises at least one T and/or T^{∗} and/or Q siloxy units. Branched organopolysiloxane can be called "resin".

According to one preferred embodiment, the branched organopolysiloxane **B** comprises at least M or M^{∗} siloxy units, at least T and/or T^{∗} , and optionally Q siloxy units. The molar ratio of (M + M^{∗})/(T + T^{∗}) can be between 0 and 1, preferably between 0 and 0.5. The molar ratio of Q/(T or T^{∗}) can be between 0 and 50%, preferably between 10% and 30%.

It can be selected from the group consisting of the silicone resins of the following formula:
- MT^{∗}, wherein the mercapto-functional groups are on the T siloxy units
- MTT^{∗}, wherein the mercapto-functional groups are on one part of the T siloxy units,
- MT^{∗}Q, wherein the mercapto-functional groups are on the T siloxy units
- MTT^{∗}Q, wherein the mercapto-functional groups are on one part of the T siloxy units,
- T^{∗}Q, wherein the mercapto-functional groups are on the T siloxy units,
- M^{∗}Q, wherein the mercapto-functional groups are on the M siloxy units,
- MM^{∗}Q, wherein the mercapto-functional groups are on one part of the M siloxy units,
- and mixtures thereof.

Branched organopolysiloxane **B** is a preferred embodiment according to the present invention. It was surprisingly found that specifically selecting a branched organopolysiloxane **B** makes it possible to manufacture 3D articles having in fine advantageous mechanical properties.

The content of mercapto function in the organopolysiloxane **B** according to the present invention is preferably between 5wt.% and 25wt.% (weight of SH, of the total weight of the organopolysiloxane **B**).

The mercapto-functional organopolysiloxane **B** according to the present invention can optionally comprise low amounts of hydroxy and/or alkoxy functions. The content of these residual hydroxy and/or alkoxy functions is preferably below 10wt.%, of the total weight of the organopolysiloxane **B.**

The mercapto-functional organopolysiloxane **B** according to the present invention can show a viscosity at 25°C preferably comprised between 10 mPa.s and 20000 mPa.s.

The content of the mercapto-functional organopolysiloxane **B** can be such that the molar ratio of mercapto function versus of alkenyl radical from the cyclic organopolysiloxane **A** or versus of (meth)acrylate radical from the cyclic organopolysiloxane **A'** is comprised between 0.1 to 10, preferably between 0.2 to 5, more preferably between 0.5 to 2. Advantageously, said molar ratio can be of around 1. It is believed that a ratio around 1 may contribute to improving the odor issue which may occur with mercapto-functional compounds. Alternatively, the content of the mercapto-functional organopolysiloxane **B** can be such that the molar ratio of mercapto function versus of alkenyl radical from the cyclic organopolysiloxane **A** or versus of (meth)acrylate radical from the cyclic organopolysiloxane **A'** can be above 1, preferably comprised between 1 to 10, more preferably between 2 to 10, or even more preferably between 5 to 10.

While the amount of the mercapto-functional organopolysiloxane **B** is adapted in view of the amount of the cyclic organopolysiloxane **A** or **A'** in order to comply with the molar ratio as defined above, the content of the mercapto-functional organopolysiloxane **B** can typically be from 5wt.% to 60wt.%, preferably from 10wt.% to 55wt.%, and even more preferably from 20wt.% to 50wt.%, of the total weight of the curable silicone composition X.

The curable silicone composition **X** according to the invention further comprises at least one photoinitiator **C.** A "photoinitiator" is a substance being able to start or initiate the curing process of a curable composition in the presence of radiation. The photoinitiator **C** can be any suitable photoinitiator well-known by the person skilled in the art such that the curable silicone composition will cure when exposed to the radiation according to the present invention.

According to a first embodiment, the photoinitiator **C** can be a type I free radical photoinitiator, preferably one compound selected from the group consisting of:
- acyl phosphine oxides, bis-acyl phosphine oxides and derivatives thereof, for example: diphenyl(2,4,6-trimethylbenzoyl)phosphine oxide (TPO), ethyl (2,4,6-trimethylbenzoyl)phenylphosphinate (TPO-L), phenyl bis(2,4,6-trimethylbenzoyl)phosphine oxide (BAPO), CPO-1 & CPO-2; 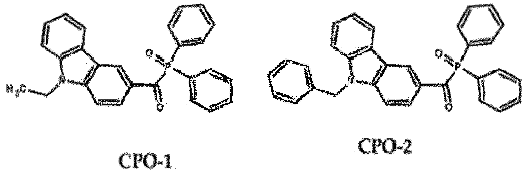
- benzoin ether;
- benzoyl oxime;
- acetophenone & hydroxyacetophenone (HAP);
- phenylglyoxate;
- alpha-hydroxyketones;
- alpha-aminoketones; and combinations thereof.

Commercial examples of type I free radical photoinitiators include: bis(2,4,6-trimethylbenzoyl)phenyl phosphine oxide (available, for example, as OMNIRAD^{™} 819, IGM Resin B.V.); liquid blends of acylphosphine oxides with at least one other photo-initiator (available, for example, as OMNIRAD^{™} 1000, OMNIRAD^{™} 2022, OMNIRAD^{™} 2100 or OMNIRAD^{™} 4265, IGM Resin B.V.); 4-(2-hydroxyethoxy)phenyl-(2-hydroxy-2-propyl) ketone (available, for example, as "OMNIRAD^{™} 2959" from IGM Resin B.V.); 2-hydroxy-2-methylpropiophenone (available, for example, as "OMNIRAD^{™} 1173" from IGM Resin B.V.); 2-benzyl-2-(N,N- dimethylamino)-1-(4-morpholinophenyl)-1-butanone (available, for example, as OMNIRAD^{™} 369 from IGM Resins B.V., or previously as Irgacure^{®} 369 from Ciba^{®}); 2- Methyl-1-[4-(methylthio)phenyl]-2-morpholinopropan-1-one (available, for example, as OMNIRAD^{™} 907 from IGM Resins B.V., or previously as Irgacure^{®} 907 from Ciba^{®}); 1-hydroxycyclohexyl benzophenone (available, for example, under the trade designation "OMNIRAD^{™} 184" from IGM Resin B.V.).

According to another embodiment, the photoinitiator **C** can be a type II free radical photoinitiator, optionally in combination with a co-initiator, preferably one compound selected from the group consisting of:
- benzophenones;
- thioxanthones, for example isopropylthioxanthone and substituted thioxanthones as disclosed in WO 2018/234643;
- 9-xanthenone;
- anthraquinones and hydroxyanthraquinones, for example: 4-dihydroxyanthraquinone, 2-methylanthraquinone, 2,2'-bis(3-hydroxy-1,4-naphthoquinone), 2,6-dihydroxyanthraquinone, 1,5-dihydroxyanthraquinone, 2-ethylanthraquinone, 2-methylanthraquinone, 1,8-dihydroxyanthraquinone ;
- 1,3-diphenyl-1,3-propanedione;
- 5,7-dihydroxyflavone;
- anthrones (i.e. anthracenones);
- phenanthrenequinone;
- camphorquinone; and combinations thereof.

The curable silicone composition **X** according to the invention can further comprise at least one photosensitizer in combination with the photoinitiator. By "photosensitizer" it is meant a molecule that absorb the energy of light and act as donors by transferring this energy to acceptor molecules. A photosensitizer can be selected among any suitable photosensitizer well-known by the person skilled in the art. Type II free radical photoinitiators can be used as photosensitizer for the type I free radical photoinitiator, especially for the type I free radical photoinitiator which do not absorb under LED light. For instance, it can be selected from the following compounds, and mixtures thereof: anthracene and substituted anthracene derivatives, pyrene, phenothiazine, Michler's ketone, xanthone(s), thioxanthone(s), benzophenone, acetophenone, carbazole derivatives, fluorenone, anthraquinone, and camphorquinone.

The photoinitiator **C** is present in the curable silicone composition **X** in a small active amount. Preferably, the curable silicone composition **X** can comprise photoinitiator **C** from 0.1% to 5% by weight, of the total weight of the composition, and preferably from 0.5% to 1.5%.

Optionally, the curable silicone composition **X** according to the invention further comprises at least one at least one filler **D.** The filler **D** can be a reinforcing filler, which can typically improve the mechanical strength of the cured silicone elastomer article.

The filler **D** can be precipitated silica, fumed (or pyrogenic) silicas, colloidal silicas and mixtures thereof. The specific surface area of these actively reinforcing fillers ought to be at least 10 m²/g, and preferably in the range from 50 m²/g to 400 m²/g, as determined by the BET method. Actively reinforcing fillers of this kind are very well-known materials within the field of the silicone rubbers. The stated silica fillers may have hydrophilic character or may have been hydrophobized by known processes.

In a preferred embodiment, the silica reinforcing filler is fumed silica with a specific surface area of at least 10 m²/g, and preferably in the range from 50 m²/g to 400 m²/g, as determined by the BET method. Fumed silica may be used as is, in an untreated form, but is preferably subjected to hydrophobic surface treatment: either a fumed silica that has been subjected to preliminary hydrophobic surface treatment may be used, or a surface treatment agent may be added during mixing of the fumed silica with the organopolysiloxane **A**, so that the fumed silica is treated in-situ.

The surface treatment agent may be selected from any of the conventionally used agents, such as alkylalkoxysilanes, alkylchlorosilanes, alkylsilazanes, silane coupling agents, titanate-based treatment agents, and fatty acid esters, and may use either a single treatment agent, or a combination of two or more treatment agents, which may be used either simultaneously or at different timings.

One example of filler is pyrogenic silica HDK^{®} H2000 commercialized by Wacker.

The amount of the silica reinforcing filler **D** in the addition-curing silicone compositions can be in the range from 0% to 50% by weight, preferably 2% to 40% by weight, more preferably 5% to 30% by weight, of the total composition.

The silicone composition X according to the invention may also comprise other fillers like a standard semi-reinforcing or packing filler. Non siliceous minerals that may be included as semi-reinforcing or packing mineral fillers can be chosen from the group constituted of: carbon black, titanium dioxide, aluminium oxide, hydrated alumina, calcium carbonate, ground quartz, diatomaceous earth, zinc oxide, mica, talc, iron oxide, barium sulfate and slaked lime.

Optionally, the curable silicone composition **X** according to the invention further comprises at least one curing inhibitor **E**. Curing inhibitors are commonly used in curable silicone compositions to slow the curing of the composition at ambient temperature. More specifically, in the method according to the present invention, the use of a curing inhibitor is effective to avoid the premature curing of the silicone composition on the tip of the nozzles and subsequent dysfunction of the printer. Additionally, a curing inhibitor can extend the shelf life of the curable composition, help prevent undesired side reactions, and adjust the polymerization process of the radiation-curable component(s) present in the curable composition. Adding one or more curing inhibitor(s) to the curable composition can further help to improving the accuracy or detail resolution of the surface of the 3D article to be produced.

The curing inhibitor **E** can be any suitable photoinitiator well-known by the person skilled in the art. Curing inhibitor(s) which can be used often comprise a phenol moiety or phosphonic acid moieties. Specific examples of curing inhibitor(s) which can be used include: p-methoxyphenol (MOP), hydroquinone monomethylether (MEHQ), 2,6-di-tert-butyl-4-methyl-phenol (BHT; Ionol), phenothiazine, 2,2,6,6-tetramethyl-piperidine-1-oayl radical (TEMPO), Vitamin E, N,N'-di-2-butyl-1,4-phenylenediamine, N,N'-di-2-butyl-1,4-phenylenediamine, 2,6-di-tert-butyl-4-methylphenol, 2,4-dimethyl-6-tert-butylphenol, 2,4-dimethyl-6-tert-butylphenol and 2,6-di-tert-butyl-4-methylphenol, 2,6-di-tert-butylphenol, pentaerythritoltetrakis(3-(3,5-di-tert-buty1-4-hydroxyphenyl)propionate) (previously known as Irganox^{™} 1010), octyl-3,5-di-tert-butyl-4-hydroxy-hydrocinnamate, octadecyl-3-(3,5-di-tert-butyl-4-hydroxyphenyl)propionate, 1,3,5-trimethyl-2,4,6-tris(3,5-di-tert-butyl-4-hydroxybenzyl)benzene, 2,2',4,4'-tetrakis-tert-butyl-3,3'-dihydroxybiphenyl, 4,4-butylidenebis(6-tert-butyl-m-cresol), 4,4'-isopropyliden-bis-(2-tert-butylphenol), 2,2'-methylenebis(6-nonyl-p-cresol), 1,3,5-tris(3,5-di-tert-butyl-4-hydroxybenzyl-)-1,3,5-triazine-2,4,6(1H,3H,5H)trione, pyrogallol, N-nitroso-N-phenylhydroxylamine, 3-propenylphenol, phenothiazine, N-phenyl-2-naphthylamine, phosphorous acid phenyl- phosphonic acid, vinylphosphonic acid or combinations or mixtures thereof.

In a preferred embodiment, the curing inhibitor is hydroquinone monomethyl ether (MeHQ).

To obtain a longer working time or "pot life", the quantity of the inhibitor is adjusted to reach the desired "pot life". When present, the concentration of the curing inhibitor **E** in the present silicone composition is preferably sufficient to slow curing of the composition at ambient temperature. This concentration will vary widely depending on the particular inhibitor used, the nature and concentration of the photoinitiator **C**, and the nature of the organopolysiloxane components. Advantageously, the amount of the curing inhibitor **E** in the curable silicone composition **X** is in the range from 0.01% to 0.2% weight, preferably from 0.03% to 0.15% weight, with respect to the total weight of the silicone composition **X.**

The silicone composition **X** according to the invention can optionally comprise other additives, for instance: a thixiotropic agent, a resin, pigments, an adhesion promoter, etc.

Advantageously, the silicone composition **X** according to the present invention does not comprise any solvent, especially it does not comprise any water or any organic solvent. The silicone composition **X** according to the present invention is not an emulsion.

In the method according to the invention, the curable silicone composition **X** has a viscosity at 70°C below 100 mPa.s, preferably below 50 mPa.s, more preferably below 20 mPa.s, and even more preferably comprised between 1 mPa.s and 20 mPa.s.

Additionally, according to a preferred embodiment, the curable silicone composition **X** has a viscosity at 20°C below 100 mPa.s, preferably below 50 mPa.s, more preferably below 20 mPa.s, and even more preferably comprised between 1 mPa.s and 20 mPa.s.

The viscosities of the silicone compositions and their individual constituents described herein correspond to a "Newtonian" dynamic viscosity magnitude at indicated temperature, i.e. the dynamic viscosity which is measured, in a manner that is known per se, with a Haak rheometer at a shear rate gradient that is low enough for the measured viscosity to be independent of the shear rate gradient. For example, the viscosity can be measured with a rheometer Haak with conic/plate 60mm geometry, by applying a sinusoidal stress of 1 Pa at 1 Hz.

The viscosity of the curable silicone composition **X** according to the invention makes it possible to easily use it with a material jetting 3D printer. Said curable silicone composition **X** can be used as an ink with a typical inkjet printing device without any complex modifications.

In a preferred embodiment, the curable silicone composition **X** of the invention comprise, with respect to the total weight of the curable silicone composition **X**:
- from 5wt.% to 70wt.% of the at least one cyclic organopolysiloxane compound **A** having between 3 and 8 silicon atoms and at least two C₂-C₈ alkenyl radicals bonded to silicon atoms per molecule, or of at least one cyclic organopolysiloxane compound **A'** having between 3 and 8 silicon atoms and at least two (meth)acrylate groups per molecule,
- from 5wt.% to 60wt.% of the at least one mercapto-containing organopolysiloxane compound **B** comprising, per molecule, at least two mercapto groups,

- from 0. 1wt.% to 5wt.% of the at least one photoinitiator **C**, and
- optionally from 0wt.% to 40wt.% of the at least one filler **D**.

In another preferred embodiment, the curable silicone composition **X** of the invention comprise, with respect to the total weight of the curable silicone composition **X**:
- from 10wt.% to 60wt.% of the at least one cyclic organopolysiloxane compound **A** having between 3 and 8 silicon atoms and at least two C₂-C₈ alkenyl radicals bonded to silicon atoms per molecule, or of at least one cyclic organopolysiloxane compound **A'** having between 3 and 8 silicon atoms and at least two (meth)acrylate groups per molecule,
- from 20wt.% to 50wt.% of the at least one mercapto-containing organopolysiloxane compound **B** comprising, per molecule, at least two mercapto groups,
- from 0.5wt.% to 1.5wt.% of the at least one photoinitiator **C**,
- from 5wt.% to 30wt.% of the at least one filler **D**, and
- from 0.01wt.% to 0.2 wt.% of a curing inhibitor **E**.

In this disclosure, "3D or three-dimensional article, object or part" means an article, object or part obtained by additive manufacturing or 3D printing as disclosed above.

The method according to the present invention provides quickly and easily 3D articles comprising at least one layer of cured silicone elastomer.

According to a first embodiment of the present invention, the 3D article is a silicone elastomer 3D article. According to one embodiment, all layers of the silicone elastomer article is produced with the same curable silicone composition. According to another embodiment, the silicone elastomer article has layers made with different curable silicone composition. This embodiment can be advantageous if an article having parts of different properties is needed.

According to a second embodiment, the 3D article obtained by the process according to the invention can be a composite article comprising one or several parts made of the cured silicone composition as defined above, and one or several parts made of another material, printed with the same 3D printer or by any other means. Indeed, material jetting 3D printer is advantageously a versatile device, and it is possible to use different types of inks. Said another material can be for instance a support suitable selected by the person skilled in the art.

The 3D article obtained by the method accord to the invention can show interesting mechanical properties. According to one embodiment, the article can show an elastic modulus below 50 MPa, preferably below 40 MPa.

Optionally, post-processing steps can improve the surface quality of the printed articles. Sanding is a common way to reduce or remove the visibly distinct layers of the model. Spraying or coating the surface of the cured material with a curable silicone composition can be used to get the right smooth surface aspect after curing it. A surfacing treatment with a laser can also be done.

For medical applications, a sterilization of the final cured material can be obtained for example: by heating either in a dry atmosphere or in an autoclave with vapor, for example by heating the object at a temperature greater than 100°C, under gamma ray, sterilization with ethylene oxide, sterilization with an electron beam.

The 3D printed article obtained by the method accord to the invention can be suitable for use in a variety of applications, especially medical applications, like medical materials and/or devices. The obtained cured material can be any article with simple or complex geometry.

Some examples are drug delivery devices, implantable devices, medical tubes, stomach catheters, medical balloons, catheter balloons, artificial dialysis machines, blood dialysis machines, implant components, chemical stoppers, O-rings, tubing in peristaltic drug delivery pumps, check valves, resuscitator bulbs, and diaphragm and prosthesis suction cups for limb attachment. Additionally, it can be for example anatomic models (functional or nonfunctional) such as heart, lung, kidney, prostate, models for surgeons and educative world or orthotics or prostheses or even implants of different classes such as long-term implants: hearing aids, stents, larynx implants, etc.

Preferably, the 3D printed article obtained by the method accord to the invention can be suitable for use as a prosthesis, more specifically as cartilage prosthesis, and even more preferably as meniscus cartilage prosthesis. Indeed, the biocompatibility of the silicone materials 3D printed by said method is high. Said material show an important resistance to thermic or chemical degradation. Moreover, the technology of material jetting 3D printing has high resolution, and therefore allows the conceptions of custom prosthesis of great accuracy.

Besides, the obtained cured material can also be an actuator for robotics, a gasket, a mechanical piece for automotive/aeronautics, a piece for electronic devices, a package for the encapsulation of components, a vibrational isolator, an impact isolator or a noise isolator.

The present invention further relates to use of a curable silicone composition **X**, which has a viscosity at 70°C of less than 100 mPa.s, and which comprises:
(A) at least one cyclic organopolysiloxane compound **A** having between 3 and 8 silicon atoms and at least two C₂-C₈ alkenyl radicals bonded to silicon atoms per molecule, or at least one cyclic organopolysiloxane compound **A'** having between 3 and 8 silicon atoms and at least two (meth)acrylate groups per molecule,
(B) at least one mercapto-containing organopolysiloxane compound **B** comprising, per molecule, at least two mercapto group,
(C) at least one photoinitiator **C**, and
(D) optionally at least one filler **D**,
   as an ink for a method for additive manufacturing of a 3D article with a material jetting 3D printer.

Additionally, the present invention relates to an ink for a material jetting 3D printer, wherein said ink consists in a curable silicone composition **X**, which has a viscosity at 70°C of less than 100 mPa.s, and which comprises:
(A) at least one cyclic organopolysiloxane compound **A** having between 3 and 8 silicon atoms and at least two C₂-C₈ alkenyl radicals bonded to silicon atoms per molecule, or at least one cyclic organopolysiloxane compound **A'** having between 3 and 8 silicon atoms and at least two (meth)acrylate groups per molecule,
(B) at least one mercapto-containing organopolysiloxane compound **B** comprising, per molecule, at least two mercapto group,
(C) at least one photoinitiator **C**, and
(D) optionally at least one filler **D**.

Various embodiments of the present invention can be better understood by reference to the following Examples which are offered by way of illustration. The present invention is not limited to the Examples given herein.

### Examples

### Materials:

POS A = 1,3,5,7-tetramethyl-1,3,5,7-tetravinyl-cyclotetrasiloxane. Viscosity at 25°C = 0.2 mPa.s.
POS B1 = a linear poly(dimethylsiloxane-co-mercaptopropyl-methyl-siloxane) with trimethylsilyl terminal groups. Viscosity at 25°C = 1500 mPa.s. Content of mercapto groups = 14wt.%
POS B2 = a branched mercapto-functional organopolysiloxane with the mean formula M₂T^{∗}₂Q_{0.6}. (T^{∗} represents the siloxy unit ((SH(CH₂)₃)SiO_{3/2}). Viscosity at 25°C = 10000 mPa.s. Content of mercapto groups = 14wt.%
POS B3 = a branched mercapto-functional organopolysiloxane with the mean formula M₂T^{∗}₂Q_{0.4}. (T^{∗} represents the siloxy unit ((SH(CH₂)₃)SiO_{3/2}). Viscosity at 25°C = 18000 mPa.s. Content of mercapto groups = 14 wt.%
PI = TPO-L (= ethyl (mesitylcarbonyl)phenyl phosphinate)
CI = MeHQ = hydroquinone monomethyl ether

The compounds were loaded in a speed mixer, then mixed during 1 minute at 2750 rpm. 3 curable silicone compositions were formulated as shown in Table 1 below:

**Table 1**

| | Ex.1 | Ex.2 | Ex.3 |
|---|---|---|---|
| POS A | 55 wt.% | 55 wt.% | 55 wt.% |
| POS B1 | 44 wt.% | | |
| POS B2 | | 44 wt.% | |
| POS B3 | | | 44 wt.% |
| PI | 1 wt.% | 1 wt.% | 1 wt.% |
| CI | 100 ppm | 100 ppm | 100 ppm |
| Molar ratio (SH/vinyl) | 1 | 1 | 1 |

The viscosity of the compositions was measured according to the following method: With a rheometer Haak with conic/plate 60mm geometry, a sinusoidal stress of 1 Pa was applied at 1 Hz to the formulation while the temperature increases of 0.1°C in Is. To evaluate the Newtonian behavior of the composition, the viscosity was measured with the same tools performing a stress sweep at 1 Hz between 1 Pa to 100 Pa. The Newtonian behavior is characterized by the invariance of the viscosity with the applied shear stress.

The curing efficiency was tested according to the following method: The compositions were poured in a photo-DSC analyzer, and irradiated at 1% power of a Hg UV light for 3mins. Time of inhibition and intensity of the peak curve were measured.

Besides, the silicone composition was poured into a mold and cured by irradiating it with a DELOLUX 20 UV lamp (400 nm) at 100% power (220 W) for 100 s. The mechanical behavior for the silicone elastomer articles after curing was tested by DMA (Dynamic Mechanical Analysis) on compression specimen. In compression mode, an increasing stress is applied to the material and the elastic modulus is taken out from the stress/strain curve.

The results are given in Table 2 below:

**Table 2**

| | Ex.1 | Ex.2 | Ex.3 |
|---|---|---|---|
| Newtonian behavior | Yes | Yes | Yes |
| Viscosity at 70°C (mPa.s) | - | 10 | 13 |
| Viscosity at 25°C (mPa.s) | 34 | 20 | 34 |
| Elastic modulus (MPa) | 70 | 33 | 29 |

## Claims

1. A method for additive manufacturing of a 3D article, said method comprising the steps of:
1) printing a first curable silicone composition on a substrate with a material jetting 3D printer and exposing to radiation to form a first layer,
2) printing a second curable silicone composition on the first or previous layer with said material jetting 3D printer and exposing to radiation to form a subsequent layer; and
3) optionally repeating step 2) with independently selected curable silicone composition for any additional layer needed;
wherein at least one of said curable silicone compositions is a curable silicone composition **X**, wherein said curable silicone composition **X** has a viscosity at 70°C of less than 100 mPa.s, and said curable silicone composition **X** comprises:
(A) at least one cyclic organopolysiloxane compound **A** having between 3 and 8 silicon atoms and at least two C₂-C₈ alkenyl radicals bonded to silicon atoms per molecule, or at least one cyclic organopolysiloxane compound **A'** having between 3 and 8 silicon atoms and at least two (meth)acrylate groups per molecule,
(B) at least one mercapto-containing organopolysiloxane compound **B** comprising, per molecule, at least two mercapto group,
(C) at least one photoinitiator **C**, and
(D) optionally at least one filler **D**.

2. The method according to Claim 1, wherein the organopolysiloxane **A** is 1,3,5,7-tetramethyl-1,3,5,7-tetravinyl-cyclotetrasiloxane.

3. The method according to claim 1 or claim 2, wherein the organopolysiloxane **B** is a polyorganosiloxane compound comprising preferably:
at least two siloxy units of formula: R_{c}Z_{d}SiO₍₄-_{c-d)/2}
wherein:
- the symbols R^{(SH)}, which may be identical or different, represent a mercapto(C₁₋₃₀)hydrocarbyl group,
- the symbols Z, which may be identical or different, represent a monovalent hydrocarbon-based group containing from 1 to 30 carbon atoms, optionally substituted by at least one halogen atom, and
- c=1, 2 or 3, preferably c=1 or 2, more preferably c=1; d=0, 1 or 2; and d+e=1, 2 or 3; and optionally other siloxy units of formula: ZₑSiO_{(4-e)/2}
wherein the symbols Z have the same definition as above, and f = 0, 1, 2, or 3.

4. The method according to claim 3, wherein the organopolysiloxane **B** is a branched organopolysiloxane.

5. The method according to any one of claims 1 to 4, wherein the content of the mercapto-functional organopolysiloxane **B** is such that the molar ratio of mercapto function versus of alkenyl radical from the cyclic organopolysiloxane **A** is comprised between 0.1 to 10, preferably between 0.2 to 5, more preferably between 0.5 to 2, and even more preferably, said molar ratio is of around 1.

6. The method according to any one of claims 1 to 5, wherein the curable silicone composition **X** comprises at least one filler **D** which is a silica reinforcing filler, more preferably fumed silica with a specific surface area of at least 50 m²/g, and preferably in the range from 100 m²/g to 400 m²/g, as determined by the BET method.

7. The method according to any one of claims 1 to 6, wherein the curable silicone composition **X** further comprises at least one curing inhibitor **E.**

8. The method according to any one of claims 1 to 7, wherein the curable silicone composition **X** has a viscosity at 70°C below 50 mPa.s, preferably below 20 mPa.s, and more preferably comprised between 1 mPa.s and 20 mPa.s.

9. The method according to any one of claims 1 to 8, wherein the curable silicone composition **X** has a viscosity at 20°C below 100 mPa.s, preferably below 50 mPa.s, more preferably below 20 mPa.s, and even more preferably comprised between 1 mPa.s and 20 mPa.s.

10. The method according to any one of claims 1 to 9, wherein the curable silicone composition **X** of the invention comprises, with respect to the total weight of the curable silicone composition **X**:
- from 5wt.% to 70wt.% of the at least one cyclic organopolysiloxane compound **A** having between 3 and 8 silicon atoms and at least two C₂-C₈ alkenyl radicals bonded to silicon atoms per molecule,
- from 5wt.% to60wt.% of the at least one mercapto-containing organopolysiloxane compound **B** comprising, per molecule, at least two mercapto groups,
- from 0. 1wt.% to 5wt.% of the at least one photoinitiator **C**, and
- optionally from 0wt.% to 40wt.% of the at least one filler **D**;
and more preferably the curable silicone composition **X** of the invention comprises, with respect to the total weight of the curable silicone composition **X**:
- from 10wt.% to 60wt.% of the at least one cyclic organopolysiloxane compound **A** having between 3 and 8 silicon atoms and at least two C₂-C₈ alkenyl radicals bonded to silicon atoms per molecule,
- from 20wt.% to 50wt.% of the at least one mercapto-containing organopolysiloxane compound **B** comprising, per molecule, at least two mercapto groups,
- from 0.5wt.% to 1.5wt.% of the at least one photoinitiator **C**,
- from 5wt.% to 30wt.% of the at least one filler **D**, and
- from 0.01wt.% to 0.2wt.% of a curing inhibitor **E**.

11. The method according to any one of claims 1 to 10, wherein the material jetting 3D printer comprises at least one printing head having a plurality of inkjet type nozzles, preferably 2 to 200 inkjet type nozzles.

12. The method according to any one of claims 1 to 11, wherein said radiation is a UV and/or a visible radiation, having preferably a wavelength between 100 nm and 450 nm, more preferable between 200 nm and 420 nm, and even more preferably between 250 nm and 405 nm.

13. A 3D printed article obtained by the method accord to any one of claims 1 to 12, for use in medical applications, like medical materials and/or devices.

14. The 3D printed article according to claim 13, for use as a prosthesis, more specifically as cartilage prosthesis, and even more preferably as meniscus cartilage prosthesis.

15. Use of a curable silicone composition X, which has a viscosity at 70°C of less than 100 mPa.s, and which comprises:
(A) at least one cyclic organopolysiloxane compound A having between 3 and 8 silicon atoms and at least two C₂-C₈ alkenyl radicals bonded to silicon atoms per molecule, or at least one cyclic organopolysiloxane compound **A'** having between 3 and 8 silicon atoms and at least two (meth)acrylate groups per molecule,
(B) at least one mercapto-containing organopolysiloxane compound **B** comprising, per molecule, at least two mercapto group,
(C) at least one photoinitiator **C**, and
(D) optionally at least one filler **D**,
as an ink for a method for additive manufacturing of a 3D article with a material jetting 3D printer.
